# EUROPÄISCHE PATENTSCHRIFT

(11) **EP 0 661 265 B1**
(45) Veröffentlichungstag und Bekanntmachung des Hinweises auf die Patenterteilung: **16.07.1997**
(21) Anmeldenummer: 94118823.7
(22) Anmeldetag: 30.11.1994
(51) Int. Cl.: C07D 211/12

(54) **Verfahren zur kontinuierlichen Herstellung von 2,2,6,6-Tetramethylpiperidin**
Process for the continuous production of 2,2,6,6-tetramethyl piperidine
Procédé pour la préparation continue de la 2,2,6,6-tétraméthyle pipéridine

(30) Priorität: 11.12.1993 DE 4342276
(43) Veröffentlichungstag der Anmeldung: 05.07.1995
(73) Patentinhaber: HOECHST AKTIENGESELLSCHAFT, 65929 Frankfurt am Main (DE)
(72) Erfinder: Kampmann, Detlef, Dr., D-86368 Gersthofen (DE); Stuhlmüller, Georg, D-86456 Gablingen (DE)

(56) Entgegenhaltungen:
- US-A- 4 208 525
- CHEMICAL ABSTRACTS, vol. 44, no. 16, 25.August 1950 Columbus, Ohio, US; abstract no. 7275d, NELSON 'studies on the mechanism of the von Braun mechanism' Spalte 1;
- CHEMICAL ABSTRACTS, vol. 56, no. 9, 30.April 1962 Columbus, Ohio, US; abstract no. 10088d, BIKOVA 'The synthesis of the ganglion-blocking preparation, Pempdin.' Spalte 1;

## Beschreibung

Die Erfindung bezieht sich auf ein kontinuierliches Verfahren zur Herstellung von 2,2,6,6-Tetramethylpiperidin durch Reduktion von 4-Oxo-2,2,6,6-tetramethylpiperidin mit Hydrazin.

2,2,6,6-Tetramethylpiperidin ist ein Produkt, das aufgrund seiner starken Basizität für die unterschiedlichsten Zwecke angewendet wird, zum Beispiel als Lichtstabilisator für Polyolefine, als Cokatalysator bei Olefinpolymerisationen (Ziegler-Katalysatoren], als Building-Block für die Synthese von Pharmawirkstoffen und Pflanzenschutzmittel, als Cokatalysator bei der Synthese von Dichloracetylchlorid, um einige Anwendungen zu nennen.

Das in 4-Stellung unsubstituierte 2,2,6,6-Tetramethylpiperidin wird im allgemeinen aus der entsprechenden 4-Oxoverbindung hergestellt.

Es ist bekannt, daß Triacetonamin mittels Hydrazin in das Hydrazon überführt werden kann, welches dann in Gegenwart von Alkali in 2,2,6,6-Tetramethylpiperidin und Stickstoff gespalten wird (vgl. CA; 44 7275 d, CA 56 10088 d). Bei der beschriebenen Reduktion mit Hydrazin wird ausschließlich diskontinuierlich gearbeitet, wobei zur Hydrazon-Spaltung große Mengen Lösemittel (ca. 11 mol Lösemittel/mol Triacetonamin) und KOH (molar 4:1) benötigt werden. Zusätzlich ist ein molarer Hydrazin-Überschuß von 1:2-1:3 nötig, um hinreichende Ausbeuten (60 bis 70 %) zu erzielen.

Dieses bisher bekannte Verfahren zur Reduktion von Triacetonamin ist jedoch für eine technische Anwendung wenig geeignet. Die Resultate stehen in keinem angemessenen Verhältnis zum Aufwand der Reduktion.

Es wurde nun gefunden, daß die Reduktion von Triacetonamin mit Hydrazin auch kontinuierlich durch eine Reaktivdestillation erfolgen kann.

Das erfindungsgemäße Verfahren wird in der Weise durchgeführt, daß zunächst aus Triacetonamin mittels Hydrazin-Hydrat das Hydrazon gebildet wird. Dazu wird Triacetonamin mit der 1,5 bis 3-molaren Menge Hydrazin-Hydrat unter Rühren gemischt. Die entstandene Lösung des Hydrazons wird am Boden einer Destillationskolonne in einen auf einer Temperatur von 160 bis 200°C gehaltenen Destillationssumpf gefördert. Dieser Destillationssumpf besteht aus einem hochsiedenden Lösemittel, welches vorwiegend OH-Gruppen enthält, wie beispielsweise ein Glycol wie Ethylenglykole, Propylenglykole, und einem Alkalihydroxid, vorzugsweise KOH. Die Hydrazon-Spaltung erfolgt unter diesen Bedingungen spontan. Neben dem freiwerdenden Stickstoff destilliert ein Gemisch, das aus 2,2,6,6-Tetramethylpiperidin, Wasser und Hydrazin besteht, über. Das Destillat trennt sich nach der Kondensation in zwei Phasen, wobei die obere Phase das 2,2,6,6-Tetramethylpiperidin mit einer Konzentration von > 90 % enthält. Die untere Phase enthält überschüssiges Hydrazin und Wasser und nur geringe Mengen des Wertproduktes.

Um eine Schaumbildung im Destillationssumpf zu vermeiden, werden 5 bis 10 % Paraffin, bezogen auf das Lösemittel, zugesetzt.

Bei dieser Verfahrensweise ist ein Hydrazin/Triacetonamin-Verhältnis von 1,5:1 bis 2,0:1 ausreichend, wobei gegenüber der herkömmlichen Synthese die Produktausbeute deutlich gesteigert werden konnte (> 90 %). Zusätzlich zu diesem Vorteil können die Einsatzmengen an Alkali und Lösemittel deutlich reduziert werden. Das Alkali/Triacetonamin-Verhältnis beträgt beim diskontontinuierlichen Verfahren 4 : 1, bezogen auf die durchgesetzte Triacetonamin-Menge, beim erfindungsgemäßen kontinuierlichen Verfahren beträgt es, ca. 1 : 40 bis 1 : 50. Die Verbesserungen beim Lösemitteleinsatz liegen in ähnlicher Größenordnung.

Eine weitere Nutzung des Destillationssumpfes zur Hydrazon-Spaltung ist problemlos möglich, wobei aber die Selektivität der Reaktion nachläßt und die Gesamtausbeute verschlechtert wird. Es wird daher vorgezogen, einen Teil des Alkali/Lösemittel-Gemisches regelmäßig zu ersetzen.

### Beispiel

In einem Rührgefäß wurde aufgeschmolzenes Triacetonamin (ca. 50 °C) vorgelegt und bezogen auf die Triacetonamin-Menge mit einer 1,5 molaren Menge Hydrazin-Hydrat versetzt. Dabei erwärmte sich die Mischung auf 80 bis 90°C. Die auf 40 bis 50°C abgekühlte Hydrazon-Lösung wurde für die nachfolgende Reaktion verwendet.

In einer Apparatur, bestehend aus einem 1 dm³-Vierhalskolben mit Vigreuxkolonne (25 cm), die mit einem Wasserabscheider und Intensivkühler versehen war, wurden 250 cm³ Triethylenglykol (= 279 g), 56,3 g Paraffinöl und 42,1 g KOH vorgelegt. Zu diesem Destillationssumpf, der unter kräftigem Rühren bei einer Temperatur von 175 bis 195°C gehalten wurde, wurden mittels einer Dosierpumpe 2,5 bis 3,5 cm³ der obigen Hydrazon-Lösung/min gepumpt. Das Hydrazon wurde unter diesen Reaktionsbedingungen spontan gespalten. Mit dem freiwerdenden Stickstoff destillierte ein Reaktionsgemisch, aus 2,2,6,6-Tetramethylpiperidin, Wasser und Hydrazin bestehend, ab. Das Kondensationsprodukt bildete zwei Phasen, die über den Wasserabscheider gemeinsam abgetrennt und anschließend separiert wurden. Die erhaltenen organischen Phasen enthielten 90 bis 95 % 2,2,6,6-Tetramethylpiperidin.

Nach einem Durchsatz von ca. 3300 g Hydrazon-Lösung ( ca. 1900 g Triacetonamin) wurde der Versuch abgebrochen. Die Analysen der organischen Phasen ergaben eine 2,2,6,6-Tetramethylpiperidin-Ausbeute von 91 %. Die vereinigten organischen Phasen wurden anschließend destillativ fraktioniert. Man erhielt ein Produkt mit einer > 99 %igen Reinheit.

## Patentansprüche

1. Verfahren zur kontinuierlichen Herstellung von 2,2,6,6-Tetramethylpiperidin durch Umsetzung von Triacetonamin mit Hydrazin und Spaltung des entstandenen Hydrazons bei einer Temperatur oberhalb 160°C, dadurch gekennzeichnet, daß das Hydrazon als wässrige Lösung kontinuierlich am Boden einer Destillationskolonne in den Destillationssumpf, welcher aus einem hochsiedenden Lösemittel **vorwiegend mit OH-Gruppen** und einem Alkali**hydroxid** besteht, gefördert wird und das entstehende 2,2,6,6-Tetramethylpiperidin als Azeotrop mit Wasser abdestilliert und vom Wasser getrennt wird.

2. Verfahren nach Anspruch 1, dadurch gekennzeichnet, daß das hochsiedende Lösemittel **ein Glykol ist**.

3. Verfahren nach Anspruch 1, dadurch gekennzeichnet, daß das **Alkalihydroxid KOH** ist.

## Claims

1. A process for the continuous preparation of 2,2,6,6-tetramethylpiperidine by reaction of triacetonamine with hydrazine and cleavage of the resulting hydrazone at a temperature of above 160°C, which comprises continuously transporting the hydrazone as an aqueous solution to the distillation bottoms which comprise a high-boiling solvent predominantly containing OH groups and an alkali metal hydroxide at the base of a distillation column and distilling off as an azeotrope with water the resulting 2,2,6,6-tetramethylpiperidine and separating it from the water.

2. The process as claimed in claim 1, wherein the high-boiling solvent is a glycol.

3. The process as claimed in claim 1, wherein the alkali metal hydroxide is KOH.

## Revendications

1. Procédé pour la préparation en continu de la 2,2,6,6-tétraméthylpipéridine par réaction de triacétonamine avec l'hydrazine et dissociation de l'hydrazone formée, à une température supérieure à 160 °C, caractérisé en ce que l'on transfère en continu l'hydrazone sous forme de solution aqueuse au fond d'une colonne de distillation dans le résidu de distillation, lequel résidu est composé d'un solvant à haut point d'ébullition, principalement contenant des groupes OH et d'un hydroxyde alcalin, et en ce que l'on distille sous forme d'azéotropes avec l'eau la 2,2,6,6-tétraméthylpipéridine formée et on sépare de l'eau.

2. Procédé selon la revendication 1, caractérisé en ce que le solvant à haut point d'ébullition est un glycol.

3. Procédé selon la revendication 1, caractérisé en ce que l'hydroxyde alcalin est KOH.
